(19) Europäisches Patentamt
European Patent Office
Office européen des brevets

(11) Publication number: 0 328 772 A2

# (12) EUROPEAN PATENT APPLICATION

(21) Application number: 88121159.3

(51) Int. Cl.4: A61K 6/06 , C04B 35/14

(22) Date of filing: 16.12.88

(30) Priority: 17.02.88 US 157206

(43) Date of publication of application:
23.08.89 Bulletin 89/34

(84) Designated Contracting States:
AT BE CH DE ES FR GB GR IT LI LU NL SE

(71) Applicant: DENTSPLY INTERNATIONAL, INC.
570 West College Avenue P.O. Box 872
York Pennsylvania 17405(US)

(72) Inventor: Pursell, Fred B., Sr.
93 Higgins Road
Locust Grove, GA 30248(US)
Inventor: Kacicz, Joseph M.
440 Ardmore Lane
York, PA 17402(US)

(74) Representative: Wächtershäuser, Günter, Dr.
Tal 29
D-8000 München 2(DE)

## (54) Dental ceramic material and methods.

(57) A porcelain paste composition and a method of building a dental appliance are provided. The paste of the invention comprises a mixture of ceramic powders and a curable adhesive resin. In the method, opaqued metal copings are coated with the paste of the invention to provide a coating thereon, and the coating is then cured. The cured coating is prepared by machining or other methods known in the art to obtain the desired shape of the coating and the coping, and the coating is then fired to fuse the ceramic powders in the coating to produce a porcelain finish on the dental appliance.

EP 0 328 772 A2

# DENTAL CERAMIC MATERIAL AND METHOD

## BACKGROUND OF THE INVENTION

A dental ceramic (porcelain) paste for applying to dental substructures, to be used in the preparation of ceramic dental appliances, such as crowns and bridges, and a method of using the same are provided.

When crowns, bridges and other metal dental substructures (copings) are to be veneered, the dental technician must first apply and fire a layer of opaque ceramic paste. This layer ensures a good bond to the metal substrate and the veneering ceramic and, in addition, masks the unfavorable metal color of the substructure which is necessary to obtain a good aesthetic appearance.

Body porcelain is applied and fired over the opaque layer. Dentin and enamel layers may also be applied and fired in due course.

Prior art ceramic paste consists of a pulverized ceramic powder which is mixed with water, or a special modeling liquid, by the dental technician. The powder itself consists of ground glass or ceramic frits, which, owing to their chemical compositions can be melted at temperatures below about 1000° C. The materials are sold in several shades since they form the color basis for the desired tooth shade.

In preparing the ceramic paste, the dental technician must adjust the consistency of the paste to obtain a paste which is sufficiently viscous so as to adhere to the opaqued metal substructure without sagging, but not so viscous that it cannot be easily applied. This procedure involves much trial and error and is very time consuming.

Prior art porcelain application requires much time and skill because a very even layer thickness has to be achieved.

The preparation of shoulder porcelain on a crown or bridge is an especially difficult application of porcelain paste. It is aesthetically desirable to provide a porcelain shoulder on a dental substructure that extends past the margin of the metal coping and below the gumline. If such a shoulder is not provided, and if the gumline recedes, the metal of the dental substructure may be exposed along the gumline causing an undesirable visual effect. Using prior art methods, the preparation of the porcelain shoulder is very difficult because of the fragile nature of a porcelain paste made with water or a similar modeling liquid. When a paste made with water is used and applied to a coping, vibrating and removal of the coping from the die may cause particles of the porcelain paste to flake off, and particles that flake off cannot be replaced (because the paste is fragile) until the remaining porcelain is stabilized, such as by firing. Because of the difficulties in application, the paste must be applied in several thin layers. Under the best of conditions in the prior art method, it may take three or more firings to apply the body porcelain alone, not making allowance for the shoulder.

Accordingly, there is a need in the art for a ceramic paste, and a method, which makes it possible to apply ceramic paste more easily and more consistently, using fewer steps, to dental substructures and whereby an even layer thickness is achieved.

## BRIEF DESCRIPTION OF THE DRAWING

FIG. 1 is an illustration of a die and coping used in making a crown according to the method of the invention and a crown made using the porcelain paste of the invention.

## SUMMARY OF THE INVENTION

The present invention provides a porcelain paste composition and a method for preparing a dental appliance.

A porcelain paste comprising ceramic powders and a curable resin modeling liquid is provided. The resin modeling liquid used in the composition may be a single component light curable liquid or a multi-component chemically cured liquid. The porcelain paste may be prepackaged in the factory. The porcelain paste may be provided in a single package or it may be provided as separate pastes in two or more separate packages.

The method comprises mixing a ceramic powder with a curable resin modeling liquid to prepare a spreadable paste, applying the paste to a dental restoration substructure to provide a coating of ceramic powder thereon, curing the resin to effectively fix the coating in its desired shape and thickness, and firing the dental restorative substructure and coating to fuse the ceramic powder. The polymerizable resin modeling liquid fluid used in the method of the invention may be a one-component or multi-component light curable resin or a self-cured (chemically cured) multi-component resin. In the firing steps, the dental restorative is fired at temperatures effective to vaporize the volatile components of the resin without burning, charring or boiling and then to fuse the ceramic powders.

Using the method and paste of the invention, it is possible for the dental technician to build a dental appliance such as a crown, inlay, onlay or a bridge using fewer steps, less time and less tedious methods. In the case where a preformed paste is provided, the paste eliminates the need for time consuming trial and error mixing of a paste in the laboratory. The assured quality control of the preformed paste that is used, and the method in which it is used lessens the possibility of applying a ceramic layer that is too thin or too thick.

## THE PRIOR ART

U. S. Patent 4,557,691, to Martin et al, teaches an opaque porcelain paste comprising an opaque porcelain powder mixed with an aqueous colloidal dispersion of urethane polymer. The paste can be applied as the first layer to a dental coping, and does not need to be fired prior to the addition of the body porcelain layers.

U.S. Patent 4,604,366 to Kacicz et al, teaches porcelain compositions, especially dental porcelain compositions, which contain leucite crystals in controlled amounts in a glassy phase matrix to permit selective adjustment of the thermal coefficient of expansion of porcelain compositions.

European Patent Application 0,119,062 teaches a storable paste-like material for use in preparing porcelain dental prostheses comprising a porcelain powder, water, and a small amount of finely divided ceramic material that overcomes the natural separation of the mixture of porcelain powder and water.

## DETAILED DESCRIPTION OF THE INVENTION

Referring now to FIG. 1, die 12, and metal coping 14 made using die 12 by casting or sintering methods known in the art, and a crown 15, made according to the method of the invention, are illustrated. Coping 14 is shown in dotted lines mounted on die 12 and as part of completed crown 15. Die 12 is made from an impression of a patient's tooth by procedures well known in the art. When coping 14 is cast or sintered, the wax mold used in the casting or sintering procedure is prepared so that the completed coping 14 will have a margin 16 which is separated from lip 17 of the die (an impression of the gumline in the illustrated embodiment) by about 0.5-1.0 mm. This 0.5-1.0 mm gap between margin 16 and lip 17 is the area that is filled with shoulder porcelain in the method of the invention to form shoulder 18. Accordingly, shoulder 18 is made to extend below margin 16 of coping 14 so that when the gumline recedes, or the edge of the crown is otherwise exposed, that the dark metal margin of the metal coping will not be seen and detract from the aesthetic effect of the crown.

The ceramic powders used in the present invention broadly constitute a known class of compositions. For most applications, the powders used in making dental ceramic pastes have a median grain size below about 50 um. Those skilled in the art will recognize that porcelain compositions having higher median grain sizes may be used depending on the application and final use of the porcelain product.

The porcelain powders used may be any commercially available ceramic powder known in the art. Exemplary of the powders that may be used are those described in U.S. Patents 4,604,366 to Kacicz et al; U.S. Patents 3,052,982; 3,052,983; and 3,400,097 to Weinstein et al and other commercially available porcelain powders sold by Dentsply International.

The ceramic composition may comprise mixtures ofa number of the following ingredients: $SiO_2$, $Al_2O_3$, $K_2O$, $Na_2O$, CaO, MgO, BaO, $Sb_2O_3$, $P_2O_5$, $CeO_2$ $Li_2O$, $ZrO_2$, $TiO_2$, F, and others as known in the art.

An illustrative ceramic composition of the invention comprises, in its ceramic powder portion, about 60-69 % $SiO_2$, about 12-20% $Al_2O_3$, about 8-14% $K_2O$, about 4.5-7.1% $Na_2O$, about 0-2% CaO, about 0-1.5% $Li_2O$, about 0-1% MgO, about 0-2.5% BaO, about 0-1.8% $Sb_2O_3$ about 0-1% $P_2O_5$, about 0-1.5% $CeO_2$,

about 0-0.5% $ZrO_2$, about 0-1% $TiO_2$, about 0-1.5% F, and about 0-1.5% $B_2O_3$.

The ceramic powder used may be combined with a polymerizable resin modeling liquid in the ratio of about 60-95% preferably 80-90% powder, and about 5-40%, preferably about 10-20% modeling liquid.

Combinations of polymerizable resins, diluents and polymerization initiators, typified by polymerizable unfilled dental resins, adhesives, and bonding agents have been found to be useful.

Any such dental polymerizable resin having properties that are compatible with ceramic powders and do not diminish the strength or appearance of the porcelain formed may be used in the method of the invention.

Particularly suitable are dental adhesives adapted to bond filling materials to teeth in dentistry, such as phosphorus containing bonding resins.

An ethylenically unsaturated polymeric monophosphate that may be used in an adhesive composition that may be used in the porcelain paste of the invention may be a polyester monophosphate, a polyether monophosphate or a polyurethane monophosphate.

In a preferred embodiment, an ethylenically unsaturated polymeric monophosphate that may be used in the invention has the formula

$$(CH_2{=}\overset{R_1}{\overset{|}{C}}{-}CO{-}O)_n\ R{-}O{-}\overset{O}{\overset{\|}{\underset{OH}{\underset{|}{P}}}}{-}OH$$

and salts thereof in which:

R is a pentaerythritol radical containing from 4 to 16 carbon atoms;

$R_1$ is a hydrogen atom, alkyl of 1 to 3 carbon atoms, halogen or -CN, and n is an integer of at least 3.

More preferred are the compositions defined above where $R_1$ is H or methyl; and n is an integer of from 3 to 6.

In the most preferred embodiments, the ethylenically unsaturated polymeric monophosphate used in the composition will be selected from the group comprising pentaerythritol triacrylate monophosphate, pentaerythritol trimethacrylate monophosphate, dipentaerythritol pentaacrylate monophosphate, dipentaerythritol pentamethacrylate monophosphate and mixtures thereof.

An adhesive composition that may be used in the invention comprises an elastomeric resin, hydroxyalkyl substituted dimethacrylate, hydroxyaryl substituted dimethacrylate, ethylenically unsaturated polymeric phosphoric acid ester, N-alkyl substituted methacrylate, hydroxy alkyl substituted methacrylate and at least one catalyst and at least one accelerator wherein alkyl is defined above.

In the preferred embodiment the adhesive composition comprises a urethane elastomeric resin, triethylene glycol dimethacrylate (TEGDMA), bisphenol-A-dimethacrylate, dipentaerythritol penta acrylate phosphoric acid ester (PENTA), 2-N-morpholinoethyl methacrylate, camphorquinone, lithium toluene sulfinate, and butylated hydroxy toluene (BHT).

The preferred urethane elastomeric resin of the invention (and the one used in the examples described below) is the reaction product of trimethyl hexamethylene diisocyanate with polytetramethylene ether glycol; capped with hydroxy ethyl methacrylate. The resin is formed using stannous octoate as a catalyst.

It is desirable, because of the edge strength required of a shoulder porcelain, that a porcelain having a higher melting range than conventional dental body porcelains be used as a shoulder porcelain in the present invention. Accordingly, to provide a porcelain having a higher melting point, a feldspar frit that has been fused and then ground up is added to a commercially available porcelain powder sold by Dentsply International.

Satisfactory porcelain powders may be obtained by combining 60-95%, preferably 80-90% commercially available porcelain powder with 5-40%, preferably 10-20% feldspar frit. A feldspar frit used in the present invention comprises about 65% $SiO_2$, about 19% $Al_2O_3$, about 13% $K_2O$, and about 3% $Na_2O$.

In the illustrated embodiment the porcelain powder composition used to prepare a porcelain shoulder on a dental restoration is a porcelain composition comprising about 80% by weight commercially available porcelain powder and about 20% by weight ground feldspar frit. Such a composition (80% by weight) was combined under yellow light with a light curable adhesive prepared according to U.S. Patent No. 4,657,941 by Blackwell et al (20% by weight) and mixed until a homogeneous paste was obtained. The paste was loaded into an opaque syringe for use in incremental amounts as needed.

Those skilled in the art will recognize that additives may be included in the composition that help

prevent separation of the porcelain powders and the modeling liquid in the syringe. For example, the porcelain powder may be loaded with a portion of ceramic powder having a particle size of less than 2 um, or may be loaded with a fumed silica. Other methods of preventing separation of the powders from the modeling liquid will be apparent to those skilled in the art.

In the method of the invention, the ceramic powder is mixed with a modeling liquid to form a ceramic paste. The paste formed from the ceramic powder and the modeling liquid is applied with a spatula to the opaqued metal substrate of a dental restorative to provide a coating thereon. In the illustrated embodiment, where the modeling liquid is a light cured one-component resin adhesive, the adhesive in the coating is then partially cured for a short period of time by directing actinic light onto the coating. The partially cured resin fixes the ceramic powder in place in the shape desired without sagging. The partially cured ceramic powder/adhesive mixture may be shaped with a knife or shaped using conventional dental grinding tools. When the porcelain paste coating is in its desired condition, the resin may be further cured using a further application of actinic light. At the convenience of the operator, the metal substrate with the ceramic powder coating is then fired. The firing temperatures are chosen in ranges that effectively vaporize the volatile components of the modeling liquid but do not cause the modeling liquid to burn, char or boil, and then fuse the ceramic powders. The modeling liquid used should not extend the firing times required. The consistency of the paste may desirably be adjusted so that it can be applied to a metal sub structure in an even layer with a spatula or other application instrument.

Using the paste of the invention as described eliminates the need for multiple application of the ceramic paste and firing steps as is common in the art.

The method of the invention makes possible the simultaneous production of a number of dental restoratives since the coating on the opaqued metal substrate, when the modeling liquid is cured, is extremely stable. The coating can be carved or machined and the restorative with the coating may be set aside indefinitely until it is desired to fire the restorative to fuse the porcelain powders. Multiple layers of coatings may be applied, and the curable resin modeling liquid therein cured, and all the layers may be fired simultaneously. Minimal shrinkage takes place on firing and the method, using the porcelain paste of the invention, produces a finished ceramic having strength and low porosity that is comparable to ceramics made according to conventional procedures.

For the convenience of the dental technician, the ceramic paste may be preformed and packaged in tubes or syringes so that required quantities can be easily extruded by the technician. Preforming the paste at the factory ensures that a paste having a specific viscosity and composition for the purpose it is to be used is consistently produced, thus avoiding the time consuming preparation of the paste in the laboratory.

The ceramic paste, as well as ceramic powders used in the method of the invention, may be provided in a number of shades. As is known in the art, various shades are produced by the addition of ceramic oxide color pigments that are known in the art.

In the case where a light-cured resin modeling liquid is used, the paste may be provided in a single package. In such an embodiment, the package will be opaque to prevent ambient light from curing the resin.

The wavelength of light used to cure the composition may be any wavelength of light that can be used safely under the circumstances of use. Generally, preferred is light having a wavelength of about 250-660 nm. More preferred is light having a wavelength of about 360-600 nm, and most preferred is light having a wavelength of about 360-520 nm.

For some applications it may be desirable to provide a porcelain paste that may be chemically cured. In such an embodiment it may be desirable to mix the resin modeling liquid and optionally accelerators with one portion of the porcelain powders to form a paste, and mix the initiators and optionally the accelerators with another portion of porcelain powder to form a second paste. The pastes can then be combined just prior to use. Such an embodiment may be desirable when an initiator or accelerator is used which is chemically incompatible over a long period of time with one or more of the components of the porcelain powder.

Similarly, in some circumstances it may be desirable to provide a light cured resin modeling liquid in two or more components where the initiators and/or accelerators are separated from each other until a multi-component paste is prepared and used. The initiators may be added in dry form to a porcelain powder and activated chemically, or using radiation or other comparable means of activation, when the porcelain powder is mixed with a resin in liquid form.

Other similar compositions for specific purposes will be apparent to those skilled in the art.

The method of the present invention has the advantages that it saves the dental technician considerable time, because application of the paste is made easier. There is less waste of material because the technique is simplified and in the case where a prepackaged paste is used, the time spent on trial and error

mixing of the paste in the laboratory is substantially eliminated. The porcelain layers may be made especially thin and even, and may be carved or ground prior to firing to assure the condition of the surface prior to firing. Also, since the technique is made simple, and more even layers are obtained, the possibility of faults developing is reduced.

It will be apparent to those skilled in the art that the ceramic paste of the invention can be used to improve substantially any method that employs a ceramic paste. Exemplary uses of the ceramic paste are to build up crowns (enamel, body, opaque, add-ons, etc.); to patch enamel (body, opaque, add-ons, etc.); to build veneers and laminates; to connect porcelain units; and to prepare inlays or onlays by direct or indirect methods.

In the direct method of preparing an inlay, an impression of a tooth cavity may be made directly using the porcelain paste of the invention, and the porcelain paste may be cured using actinic light while in the cavity. The cured porcelain paste can then be removed from the cavity and fired in a ceramic furnace to fuse the porcelain powders therein. The prepared porcelain inlay can then be secured in the cavity using a dental cement.

Other uses of the porcelain paste of the invention will be apparent to those skilled in the art.

For convenience, the materials used in the methods of the invention may be provided in kit form.

In addition to a prepackaged porcelain paste, the kit may be provided with additional ceramics for use in the opaque layers, body layers and the dentin and enamel layers and other conventional materials as required. To the degree possible, these materials may be provided in a preformed paste, or in conventional states. For example, a preformed ceramic opaque paste may be made using a dental adhesive or other modeling liquids known in the art.

The method of the invention and the properties of the product obtained are illustrated by the following examples.

## EXAMPLE 1

The resin used in a preferred embodiment of the resin modeling liquid used to make the porcelain paste of the invention is made in accordance with the following:

### Preparation of PENTA

Dipentaerythritol Pentaacrylate Phosphoric Acid Ester (PENTA).

A solution of technical dipentaerythritol monohydroxypentaacrylate (1 mole) and triethylamine (1 mole) in dry ether was slowly added with stirring to a solution of phosphorus oxychloride (1 mole) in dry ether, at 0° C. After stirring for two hours at room temperature, the reaction mixture to ice water with stirring at below 10° C. The resultant mixture was separated and the separated ether layer was then extracted with a 25% aqueous sodium carbonate solution. The aqueous extract exhibited a pH of about 8. The alkaline aqueous extract was then acidified with 18% hydrochloric acid and an oily material was formed. The oily material was extracted with methylene chloride and the extract was dried over anhydrous sodium sulphate. The methylene chloride was then removed from the dried extract under reduced pressure to give the title compound as a clear straw-colored oil.

### Preparation of Urethane Dimethacrylate (UDMA)

To a mechanically-stirred mixture of 28.59 weight parts of trimethylhexamethylene diisocyanate and 0.04 weight part of stannous octoate in a reactor and purged with dry air, there is added 44.80 weight parts of polytetramethylene ether glycol, while maintaining the reaction temperature at 50° to 60° C. After the addition is complete, the mixture is heated to 70° to 80° C and held for 3 hours. A 26.58 weight parts of hydroxyethyl methacrylate (HEMA) was added and the mixture was stirred at 70° to 80° C until the percent NCO assay was below 0.01%.

Preparation of Curable Resin Modeling Liquid

A mixture of 55.44 weight parts of UDMA, 27.77 weight parts of TEGDMA, 9.6 weight parts or Bis-phenol A Dimethacrylate and 0.02 weight part of butylated hydroxy-toluene was heated in a reactor at 40° to 45° C until a homogenous mixture was obtained. To this mixture, 4.73 weight parts of PENTA and 2.08 weight parts of N-morpholinoethyl methacrylate were added and mixed for 1/2 hour at 40° to 45° C. A mixture of 0.12 weight part of lithium p-toluene-sulfinate and 0.24 weight part of camphoroquinone was added to the above mixture and stirred for 1 hour at 40° to 45° C.

EXAMPLE 2

As an example of the composition that has been used for the shoulder porcelain, the following is provided which is the resultant composition of combining about 80% Biobond®, a ceramic powder sold by Dentsply International, with about 20% feldspar frit as described above.

| | WT % |
|---|---|
| $SiO_2$ | 63.8 |
| $Al_2O_3$ | 16.3 |
| $K_2O$ | 11.2 |
| $Na_2O$ | 5.2 |
| CaO | 0.6 |
| $Li_2O$ | 0.2 |
| BaO | 1.2 |
| $Sb_2O_3$ | 0.1 |
| $B_2O_3$ | 0.4 |
| $TiO_2$ | 0.2 |
| $ZrO_2$ | 0.2 |
| $CeO_2$ | 0.6 |
| | 100.0 |

All oxides are ingredients of ground ceramic frits.

Depending on the raw materials used, analyses of a particular batch of Biobond® may also show small amounts of F, MgO, $Fe_2O_3$, and other compositions common in porcelain powders.

The above ceramic powder (80% by weight) was combined under yellow light with a light curable adhesive prepared according to Example 1 and mixed until a homogeneous paste was obtained. The paste was loaded into an opaque syringe for use in incremental amounts as needed.

EXAMPLE 3

A model of tooth die and a dental casting (coping) for a tooth crown were prepared as is conventional in the art. The margin of the coping was modified as is customary in the art to accommodate a porcelain shoulder. The metal substructure was opaqued according to known procedures in the art and fired. The margin area of the die was painted with a die sealant and allowed to dry.

After the die sealant was completely dried, the margin area of the coping and the lip area of the die was painted with one coat of porcelain release (Al-Cote® or Spectrum™ shoulder separator, products of Dentsply International). The porcelain release makes easy the separation of the coping from the die after porcelain layers have been added.

One drop of polymerizable resin modeling liquid of Example 1 was added to 1/2 scoop of the appropriately shaded shoulder porcelain powder of Example 2 and the powder and modeling liquid were mixed to a doughy consistency. The metal casting was placed on the die and the doughy mix of porcelain powder and modeling liquid was applied to the opaqued metal substructure and the margin area of the die using a spatula or other dental instrument. The shoulder porcelain was placed and packed on the die while

making sure that margin porcelain was not extended into any undercuts. The coated die, and coping were vibrated to condense the paste and the paste was blotted to remove exuded modeling liquid from the surface of the paste. The marginal porcelain was extended slightly up onto the opaqued metal to ensure that the shoulder would adhere to the metal when the shoulder was cured.

The marginal area of the die (the porcelain shoulder of the metal coping) was cured for about 10 seconds using a Prisma Lite® light curing apparatus, a product of L.D. Caulk, a division of Dentsply International. Ten seconds is considered sufficient for a partial cure of the adhesive in the shoulder porcelain.

The coping was replaced on the die and additional porcelain, from a prepackaged syringe, to illustrate the use of prepackaged porcelain paste, was added to the coping as needed to further smooth and contour the surface of the paste covered coping. The shoulder was then further cured using an additional ten seconds exposure to Primsa Lite®. The cured paste was then ground, using a conventional dental grinding tool to further smooth the surface of the cured porcelain paste.

The coping together with the shoulder porcelain was lifted off the die and the inside of the coping was examined for excess porcelain paste on the inside of the coping. Since the curing light did not reach the inside of the coping to cure the porcelain paste, the excess porcelain was easily removed using a bladed instrument. The cured shoulder and porcelain face was shaped and smoothed as desired using a sharp bladed dental instrument.

The metal coping, with the porcelain coating and shoulder was placed on a firing tray point and placed near the porcelain furnace for five minutes to dry, preheat and initiate the removal of volatile portions of the modeling liquid.

The firing tray point with the paste covered coping was then placed in the porcelain furnace for ten minutes at about 600° C to complete the dryout procedure. Thereafter the coping was program fired up to a temperature of about 985° C over a five minute period, the furnace being programmed to rise in temperature about 100° C/minute during the programmed firing.

The fired coping, after inspection, was found to be in good form without the need for further work.

If further application of shoulder porcelain is desired, it has been shown that further porcelain can be added and the coping refired until a desirable product is obtained. No loss of stability was observed in one instance in which the substructure was fired seven times.

EXAMPLE 4

It has been found that dental adhesive can also be used to repair chips in the margin.

A coping having a chipped margin where metal was exposed was repaired by mixing dental opaque with dental adhesive. The opaque was applied and fired to cover the exposed metal, and subsequently, porcelain was applied and fired in the same manner as described above.

EXAMPLE 5

The strength of the shoulder porcelain of a completed coping was tested using the thermal shock test.

A completed coping was heated to about 940° C and placed directly into cold water. It was observed that the shoulder porcelain was more resistant to shatter than the porcelain layered directly over the metal coping.

EXAMPLE 6

The strength of the porcelain made using the porcelain paste and method of the invention was tested as follows:

MOR (Modulus of Rupture) bars were prepared by mixing a batch of porcelain paste on a glass plate, loading 1/8" glass tubing with the paste by pushing the bottom of the glass tubing down into the paste, and packing the paste from the top of the tube using a glass rod. The paste was light cured for 90 seconds in the tubing using a TRIAD II® light curing apparatus, a product of Dentsply International, and then placed in

a freezer. On removal from the freezer, the cured rod of material was separated from the glass tubing by pushing the material out of the tubing using a glass rod. The rod of material was then warmed from room temperature up to about 1100° F in a ceramic furnace to remove volatile materials. The porcelain was then fired up to about 1800° F over a five minute interval.

The mean value of the MOR for six bars so formed and subjected to an INSTRON constant rate loading machine was about 11,000 psi (pounds per square inch).

While present embodiments of the invention have been illustrated and described, it will be recognized by those skilled in the art that the present invention may be otherwise variously embodied and practiced without departing from the scope of the following claims.

## Claims

1. A porcelain paste composition comprising ceramic powder and polymerizable resin modeling liquid in which said resin is selected from the group comprising a single component light polymerizable resin, multi-component light polymerizable resin, and multi-component chemically cured resin and said ceramic powder comprises about 60-69% $SiO_2$, about 12-20% $Al_2O_3$, about 8-14% $K_2O$, about 4.5-7.1% $Na_2O$, about 0-2% CaO, about 0-1.5% $Li_2O$, about 0-1% MgO, about 0-2% 5% BaO, about 0-1.8% $Sb_2O_3$, about 0-1 % $P_2O_5$, about 0-1.5% $CeO_2$, about 0-0.5% $ZrO_2$, about 0-1% $TiO_2$, about 0-1.5% F, and about 0-1.5% $B_2O_3$.

2. The composition of claim 1 in which said resin is a dental adhesive adapted to bond filling material to teeth.

3. The composition of claim 1 in which said resin comprises a phosphorus containing polymerizable methacrylate resin.

4. The composition of claim 1 in which said ceramic powders comprise 5-40% by weight ground feldspar frit.

5. The composition of claim 2 in which the adhesive composition comprises an elastomeric resin, hydroxyalkyl substituted dimethacrylate, hydroxyaryl substituted dimethacrylate, ethylenically unsaturated polymeric phosphoric acid ester, N-alkyl substituted methacrylate, hydroxy alkyl substituted methacrylate and at least one catalyst and at least one accelerator.

6. The composition of claim 2 in which said curable resin adhesive comprises an ethylenically unsaturated polymeric monophosphate of the formula

$$(CH_2{=}\overset{R_1}{\underset{}{C}}{-}CO{-}O)_n\ R{-}O{-}\overset{O}{\underset{OH}{P}}{-}OH$$

and salts thereof in which:

R is a pentaerythritol radical containing from 4 to 16 carbon atoms;

$R_1$ is a hydrogen atom, alkyl of 1 to 3 carbon atoms, halogen or -CN, and n is an integer of at least 3.

7. The composition of claim 1 in which said ceramic powder comprises

| | WT % |
|---|---|
| $SiO_2$ | 63.8 |
| $Al_2O_3$ | 16.3 |
| $K_2O$ | 11.2 |
| $Na_2O$ | 5.2 |
| CaO | 0.6 |
| $Li_2O$ | 0.2 |
| BaO | 1.2 |
| $Sb_2O_3$ | 0.1 |
| $B_2O_3$ | 0.4 |
| $TiO_2$ | 0.2 |
| $ZrO_2$ | 0.2 |
| $CeO_2$ | 0.6 |
| | 100.0 |

8. The composition of claim 2 in which said curable resin adhesive comprises urethane elastomeric resin, triethylene glycol dimethacrylate (TEGDMA), bisphenol-A-dimethacrylate, dipentaerythritol penta acrylate phosphoric acid ester (PENTA), 2-N-morpholinoethyl methacrylate, camphorquinone, lithium toluene sulfinate, and butylated hydroxy toluene (BHT).

9. A method for making a dental restorative comprising the steps of:

(a) Mixing ceramic powder with a polymerizable resin modeling liquid to prepare spreadable paste;

(b) Applying said paste to dental restorative substructure to provide a coating of ceramic paste thereon;

(c) Curing said resin to effectively fix said coating in its desired shape and thickness; and

(d) Firing said dental restorative substructure and said coating to fuse said ceramic powder.

10. The method of claim 9 in which said resin is a dental adhesive adapted to bond filling material to teeth.

11. The method of claim 9 wherein said resin is phosphorus containing methlymethacrylate.

12. The method of claim 9 comprising firing said dental restorative in a manner and at a temperature in which said resin is first vaporized without burning, charring or boiling and then said ceramic powder is fused.

13. The method of claim 9 in which said ceramic powder comprises about 60-69 % $SiO_2$, about 12-20 % $Al_2O_3$, about 8-14 % $K_2O$, about 4.5-7.1% $Na_2O$, about 0-2% CaO, about 0-1.5% $Li_2O$, about 0-1% MgO, about 0-2. 5% BaO, about 0-1.8% $Sb_2O_3$, about 0-1% $P_2O_5$, about 0-1.5% $CeO_2$, about 0-0.5% $ZrO_2$, about 0-1% $TiO_2$, about 0-1.5% F, and about 0-1.5% $B_2O_3$.

14. The method of claim 9 in which said ceramic powder comprises about 5-40% by weight ground feldspar frit.

15. The method of claim 9 in which said polymerizable resin comprises a compound of the formula

$$(CH_2{=}\overset{R_1}{C}{-}CO{-}O)_n\ R{-}O{-}\overset{O}{\underset{OH}{P}}{-}OH$$

and salts thereof in which:

R is a pentaerythritol radical containing from 4 to 16 carbon atoms;

$R_1$ is a hydrogen atom, alkyl of 1 to 3 carbon atoms, halogen or -CN, and n is an integer of at least 3.

16. The method of claim 9 in which said adhesive composition comprises an elastomeric resin, hydroxyalkyl substituted dimethacrylate, hydroxyaryl substituted dimethacrylate, ethylenically unsaturated polymeric phosphoric acid ester, N-alkyl substituted methacrylate, and at least one catalyst and at least one accelerator.

17. The method of claim 9 in which said ceramic powder comprises

| | WT % |
|---|---|
| $SiO_2$ | 63.8 |
| $Al_2O_3$ | 16.3 |
| $K_2O$ | 11.2 |
| $Na_2O$ | 5.2 |
| CaO | 0.6 |
| $Li_2O$ | 0.2 |
| BaO | 1.2 |
| $Sb_2O_3$ | 0.1 |
| $B_2O_3$ | 0.4 |
| $TiO_2$ | 0.2 |
| $ZrO_2$ | 0.2 |
| $CeO_2$ | 0.6 |
| | 100.0 |

18. The method of claim 10 in which said curable resin adhesive comprises a urethane elastomeric resin, triethylene glycol dimethacrylate (TEGDMA), bisphenol-A-dimethacrylate, dipentaerythritol penta acrylate phosphoric acid ester (PENTA), 2-N-morpholinoethyl methacrylate, camphorquinone, lithium toluene sulfinate, and butylated hydroxy toluene (BHT).

19. A dental restorative made according to the method of claim 9.

20. The use of the ceramic paste of claim 1 for preparing a dental restorative.

21. The use of the ceramic paste of claim 1 for building up crowns.

22. The use of the ceramic paste of claim 1 for patching enamel.

23. The use of the ceramic paste of claim 1 for building veneers and laminates.

24. The use of the ceramic paste of claim 1 for connecting porcelain bridgework units.

25. The use of the ceramic paste of claim 1 for preparing direct and indirect inlays and onlays.

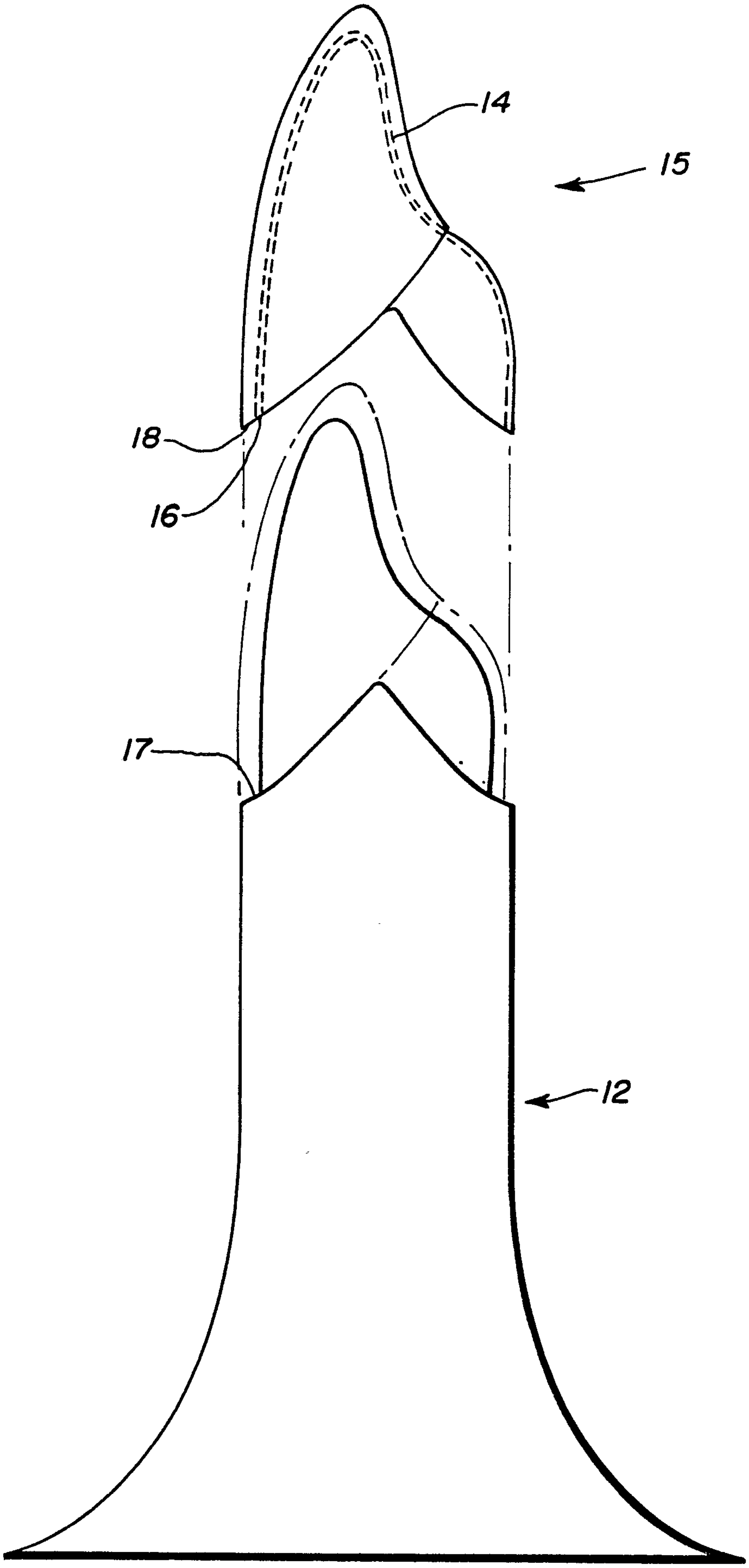


Fig. 1